# EUROPÄISCHE PATENTSCHRIFT

(11) **EP 3 062 727 B1**
(45) Veröffentlichungstag und Bekanntmachung des Hinweises auf die Patenterteilung: **09.03.2022**
(21) Anmeldenummer: 14792526.7
(22) Anmeldetag: 31.10.2014
(51) Int. Cl.: A61B 90/98, A61B 34/20, A61B 90/00, A61B 17/00

(54) **CHIRURGISCHES INSTRUMENT UND LAGEERFASSUNGSSYSTEM ZUR LAGEERFASSUNG EINES CHIRURGISCHEN INSTRUMENTS**
SURGICAL INSTRUMENT AND SYSTEM FOR DETECTING THE POSITION OF A SURGICAL INSTRUMENT
INSTRUMENT CHIRURGICAL ET SYSTÉME DE DÉTECTION DE POSITION D'UN INSTRUMENT CHIRURGICAL

(30) Priorität: 31.10.2013 DE 102013222230
(43) Veröffentlichungstag der Anmeldung: 07.09.2016
(73) Patentinhaber: Intersect ENT International GmbH, 16761 Hennigsdorf (DE)
(72) Erfinder: KRÜGER, Timo, 13465 Berlin (DE); MUCHA, Dirk, 13467 Berlin (DE)
(74) Vertreter: Eisenführ Speiser
(86) Internationale Anmeldenummer: PCT/EP2014/073493
(87) Internationale Veröffentlichungsnummer: WO 2015/063280

(56) Entgegenhaltungen:
- EP-A1- 2 305 115
- WO-A1-2012/150567
- WO-A1-2013/109527
- DE-A1-102010 027 535
- US-A1- 2003 069 588

## Beschreibung

Die Erfindung betrifft ein chirurgisches Instrument mit einer Lokalisatoranordnung zur verbesserten Bestimmung der Lage der Instrumentenspitze in einem Lageerfassungssystem. Ein Verfahren zur Lageerfassung eines chirurgischen Instruments ist ebenfalls offenbart.

Ein Lageerfassungssystem ist ein System zur Bestimmung der Lagedaten - nämlich der Position und Orientierung - des Arbeitspunkts eines medizinischen Instruments, wie z.B. eines Sichelmessers, Antrumlöffels, Pointers, Skalpells, Elektrotoms oder Käufers, innerhalb eines Referenzkoordinatensystems. Der Arbeitspunkt eines medizinischen Instruments ist z.B. das distale Ende einer Instrumentenspitze. Ein Referenzkoordinatensystem ist ein Koordinatensystem einer Lageerfassungseinrichtung bzw. eines Lageerfassungssystems. Mit Hilfe der Lageinformationen und Daten über Form und Dimension des Medizinischen Instruments ist das medizinische Instrument lagegetreu in dem Referenzkoordinatensystem auf einer Anzeigeeinheit darstellbar. Mit Hilfe des Lageerfassungssystems ist die lagegetreue Abbildung des medizinischen Instruments mit einer lagegetreuen Abbildung des jeweiligen Körperteils des Patienten überlagert darstellbar.

Für die Planung und Durchführung invasiver chirurgischer Eingriffe ist es bekannt, als Planungsdaten präoperativ gewonnene Bilddaten eines Operationsgebiets bzw. Eingriffsgebiets im Inneren eines Patienten, wie z.B. Röntgen-, Sonographie-, Computertomographie- (CT) oder Magnetresonanztomographieaufnahmen (MRT) zu verwenden. Das Eingriffsgebiet ist der Bereich im und am Körper des Patienten, in dem während des operativen Eingriffs die operative Maßnahme, wie z.B. das Abtrennen eines Gewebeteils, das Verschließen eines Gefäßes oder das Einsetzen eines Implantats, durchgeführt wird, während das Operationsgebiet das gesamte Gebiet am und im Körper des Patienten ist, das während des operativen Eingriffs beeinträchtigt wird, z.B. auch durch das bloße Entlangführen von chirurgischen Instrumenten.

Des Weiteren ist es üblich, intraoperativ gewonnene Bilddaten, z.B. endoskopische Livevideo- oder CT-Aufnahmen, auf einer Anzeigeeinheit, wie z.B. einem Monitor, zusammen mit den präoperativ gewonnenen Bilddaten nebeneinander oder überlagert darzustellen. Auf diese Weise können beispielsweise zu entfernendes Gewebe, wie z.B. Tumore, sowie im Operationsgebiet liegende und durch den operativen Eingriff potenziell gefährdete, sensible Objekte, wie z.B. Nerven(-bahnen), Muskeln, Sehnen, Organe oder Gefäße, graphisch hervorgehoben und dem Operateur somit besser sichtbar gemacht werden. Auf diese Weise hilft das Lageerfassungssystem dem Operateur, die bei der Operation verwendeten medizinischen Geräte möglichst effizient und unter minimaler Beeinträchtigung des umliegenden Gewebes des Patienten einzusetzen. Da das Lageerfassungssystem bereits die Lagedaten des medizinischen Geräts in das Referenzkoordinatensystem transformiert, entfällt für den Operateur ein entsprechender Schritt, so dass er sich im Wesentlichen auf die einzelnen Schritte der durchzuführenden Operation konzentrieren kann. Hierdurch können die Dauer der Operation sowie das durch die Operation entstehende Gesundheitsrisiko für den Patienten erheblich reduziert werden.

Während der Durchführung einer Operation muss zunächst das medizinische Instrument möglichst genau entlang einem vorgegebenen Weg in das meist präoperativ festgelegte Eingriffsgebiet im Körper des Patienten navigiert werden, um benachbartes Gewebe nicht zu beschädigen. Hierbei kann sich der Operateur im Wesentlichen auf die überlagerte Darstellung des chirurgischen Instruments mit den Bilddaten des Operationsgebiets des Patienten sowie die Führung des chirurgischen Instruments konzentrieren. Im Eingriffsgebiet führt der Operateur die operative Maßnahme möglichst präzise durch, um einerseits das Operationsziel, z.B. vollständiges Entfernen eines vorbestimmten Gewebeteils oder vollständiges Verschließen eines Gefäßes, zu erreichen und andererseits dabei möglichst wenig umliegendes, gesundes Gewebe zu beschädigen. Nach Beendigung der operativen Maßnahme muss das medizinische Instrument wieder kontrolliert und präzise entlang einem vorgegebenen Weg aus dem Eingriffsgebiet und aus dem Operationsgebiet herausgeführt werden.

Hierfür sind eine Vielzahl unterschiedlicher Positionserfassungs- bzw. Lageerfassungssysteme bekannt, die eine Navigation von medizinischen Instrumenten, wie z.B. chirurgischen Instrumenten, im Operationsgebiet im und am Körper des Patienten unterstützen. Diese Systeme erfassen während der Operation die Koordinatentransformation zwischen dem Patienten und mindestens einem medizinischen Instrument z.B. kontinuierlich oder intermittierend. Ferner sind Lageerfassungssysteme bekannt, mit denen die Lage einer Vielzahl von unterschiedlichen medizinischen Instrumenten erfassbar sowie in das Referenzkoordinatensystem übertragbar sind. Die ermittelten Lagedaten werden in der Regel zusammen mit den präoperativ gewonnenen Planungsdaten und/oder den intraoperativ gewonnenen Bilddaten auf einer Anzeigeeinheit dargestellt.

Damit Lageerfassungssysteme die Lage von medizinischen Instrumenten sowie dem Patienten erfassen können, werden Lokalisatoren eingesetzt, die an den medizinischen Instrumenten und dem Patienten angebracht werden. Üblicherweise sind Lokalisatoren bereits in den entsprechenden medizinischen Instrumenten integriert, während ein Patientenlokalisator, z.B. in Form eines Stirnbands, vor der Operation erst am Patienten befestigt werden muss. Mit Hilfe der Lokalisatoren sind zunächst die Lagen der einzelnen Lokalisatoren im Referenzkoordinatensystem vom Lageerfassungssystem bestimmbar.

Ein Lokalisator kann eine bestimmte Anzahl von Freiheitsgraden erfassen. Zur vollständigen Bestimmung der Lage eines dreidimensionalen Objekts, wie z.B. eines chirurgischen Instruments, in einem dreidimensionalen Raum mit den kartesischen Achsen X, Y, Z sind sechs Freiheitsgrade zu ermitteln, nämlich drei translatorische (X'-,Y'-,Z'- Komponente) sowie drei rotatorische (Rotation um X'-, Y'-, Z'-Achse). Es gibt Lokalisatoren, die ausgebildet sind alle sechs Freiheitsgrade erfassen. Andere Lokalisatoren können beispielsweise nur fünf oder weniger Freiheitsgrade erfassen. Eine eindeutige Lagebestimmung eines Objekts mit einem Lokalisator, der zur Erfassung von weniger als sechs Freiheitsgraden ausgebildet ist, ist demnach nicht möglich. Dafür können mehrere Lokalisatoren miteinander kombiniert werden, um gemeinsam alle zur eindeutigen Lagebestimmung erforderlichen Freiheitsgrade zu erfassen.

Des Weiteren gibt es unterschiedliche Arten von Lokalisatoren, wie z.B. optische, akustische oder elektromagnetische Lokalisatoren.

Optische Lokalisatoren weisen Reflektoren oder optische Markerpunkte auf, deren Abbildung in einer Aufnahme möglichst leicht und eindeutig zu identifizieren ist. Die Abbilder der optischen Lokalisatoren können von einer Auswerteeinheit fotogrammetrisch ausgewertet werden, um somit die Lage des optischen Lokalisators im Referenzkoordinatensystem zu bestimmen. Anstelle dezidierter optischer Lokalisatoren kann eine optische Lageerkennung auch anhand charakteristischer Form eines jeweiligen Instruments erfolgen. Ggf. ist dann ein etwas höherer Auswertungsaufwand erforderlich. In diesem Sinne können auch charakteristische Formen eines Instruments einen Lokalisator zum Bestimmen aller sechs Freiheitsgrade mittels eines optischen Lagererfassungssystems bilden. Optische Lageerfassungssysteme haben den Vorteil, dass sie kaum elektromagnetische Störfelder emittieren und auch bei starken elektromagnetischen Störfeldern keine Messfehler produzieren. Nachteilig ist, dass optische Lokalisatoren relativ zu elektromagnetischen Lokalisatoren eine größere Bauform aufweisen und nur funktionieren, wenn keine Hindernisse zwischen Lichtquelle bzw. Empfangseinheit und Lokalisator angeordnet sind.

Elektromagnetische Lageerfassungssysteme haben sich insbesondere wegen der guten Integrierbarkeit der elektromagnetischen Lokalisatoren in medizinische Instrumente und ihrer hohen Zuverlässigkeit bewährt. Elektromagnetische Lageerfassungssysteme weisen einen Feldgenerator auf, der z.B. als Kopfkissen für den Patienten ausgebildet oder neben dem Patienten angeordnet ist. Der Feldgenerator emittiert ein alternierendes elektromagnetisches Feld. Die Lokalisatoren eines elektromagnetischen Lageerfassungssystems weisen Spulen auf, die mit einer Auswerteeinheit des Lageerfassungssystems verbunden sind. In Abhängigkeit der Ausrichtung und Position der Spulen im alternierenden elektromagnetischen Feld wird in jeder Spule ein elektrischer Strom induziert. Aufgrund der Stromstärke kann die Auswerteeinheit die genaue Lage des Lokalisators relativ zum Feldgenerator bestimmen. Da die Position des Feldgenerators im Referenzkoordinatensystem bekannt ist, ist somit die Lage des jeweiligen Lokalisators im Referenzkoordinatensystem bekannt.

Damit auch die Lage des Arbeitspunkts einer Instrumentenspitze im Referenzkoordinatensystem bestimmbar ist, muss das entsprechende Instrument zunächst im Lageerfassungssystem registriert bzw. eingemessen werden. Hierfür wird mit dem Arbeitspunkt des medizinischen Instruments ein im Referenzkoordinatensystem bekannter Referenzpunkt, z.B. ein Punkt auf dem Patientenlokalisator, angefahren. Über die Lage des Lokalisators des Instruments und die Lage des Referenzpunkts im Referenzkoordinatensystem vom Lageerfassungssystem kann das Lageerfassungssystem den Richtungsvektor des Abstands des Arbeitspunkts relativ zum Lokalisator des Instruments bestimmen. Mittels einer Instrumentendatenbank kann das Lageerfassungssystem eine lagegetreue Abbildung des entsprechenden Instruments auf der Anzeigeeinheit erzeugen.

Des Weiteren muss auch der Patient in dem Lageerfassungssystem registriert werden. Hierfür wird zunächst der Patientenlokalisator am Patienten angebracht. Mittels eines Pointers, dessen Abtastspitze im Lageerfassungssystem registriert ist, werden Referenzpunkte am Körper des Patienten angefahren. Als Referenzpunkte sind besonders Körperteile geeignet, bei denen zwischen Haut und Knochen wenig Gewebe vorhanden ist, wie z.B. Gelenke. Hierdurch wird die Genauigkeit des Registrierungsprozesses des Patienten verbessert. Bei Erreichen eines Referenzpunkts kann manuell das Erreichen des jeweiligen Punkts bestätigt werden, so dass das Lageerfassungssystem die Koordinaten des Referenzpunkts über die Lagedaten des Pointers ermitteln und anschließend speichern kann. In einigen Systemen reicht ein kurzes Verweilen an dem Referenzpunkt, um einen automatischen Ermittlungs- und Speicherprozess der Koordinaten des Referenzpunkts zu initiieren. Je mehr Referenzpunkte angefahren werden, desto genauer kann das Lageerfassungssystem Präoperativ und/oder intraoperativ gewonnene Bilddaten des Patienten in das Referenzkoordinatensystem übertragen.

Medizinische Instrumente sind entweder wiederverwertbare Instrumente, sogenannte "multiple-use" Instrumente, die vor bzw. nach jedem Einsatz zu sterilisieren sind, um das Infektionsrisiko für den Patienten zu reduzieren, oder Einweginstrumente, sogenannte "single-use" Instrumente, die in der Regel steril verpackt sind und nach Gebrauch entsorgt werden müssen.

US2003/0069588 A1 offenbart ein chirurgisches Instrument mit einem Instrumentengriff, einem mit dem Instrumentengriff verbundenen Instrumentenschaft, einer mit dem Instrumentenschaft verbundenen Instrumentenspitze mit einem Arbeitspunkt, sowie einem an dem Instrumentengriff oder dem Instrumentenschaft angeordneten ersten Lokalisator. Der erste Lokalisator ist ausgebildet, sechs Freiheitsgrade zu erfassen. Der erste Lokalisator ist weiter als Lagesensor ausgebildet und weist zwei Spulen auf. Der Instrumentenschaft ist auslenkbar zwischen dem ersten Lokalisator und dem Arbeitspunkt während eines Einsatzes des chirurgischen Instruments. Das Instrument beinhaltet einen zweiten Lokalisator mit Abstand zum ersten Lokalisator und gegenüber diesem näher an dem Arbeitspunkt, wobei der zweite Lokalisator ausgebildet ist, nur fünf Freiheitsgrade zu erfassen und als Lagesensor ausgebildet ist und eine Spule aufweist. Der zweite Lokalisator weist kleinere Abmessungen als der erste Lokalisator auf.

Herkömmliche Lageerfassungssysteme haben den Nachteil, dass der Einsatz von medizinischen Instrumenten, die relativ flexiblen Bereich zwischen ihrem Arbeitspunkt und dem Lokalisator aufweisen, zu Fehlbestimmungen des Arbeitspunkts führt, wenn der Arbeitspunkt wegen eines Widerstands, z.B. des Gewebes des Patienten, aus seiner natürlichen Position relativ zum Lokalisator des medizinischen Instruments ausgelenkt wird. Hierbei wird der flexible Instrumentenschaft meist elastisch verformt. Wenn der Widerstand nachlässt, federt der Arbeitspunkt wieder in seine ursprüngliche Position relativ zum Lokalisator zurück. Die herkömmlichen Lageerfassungssysteme sind nicht in der Lage, die Durchbiegung des Schaftteils zwischen dem Lokalisator und dem Arbeitspunkt zu kompensieren und ermitteln daher Koordinaten eines (fiktiven) Arbeitspunkts, bei nicht gebogenem Instrumentenschaft. Um eine solche Fehlbestimmung möglichst gering zu halten, ist der Lokalisator bei medizinischen Instrumenten mit flexiblem Schaft möglichst nah an dem Arbeitspunkt der Instrumentenspitze angeordnet. Ein Mindestabstand zwischen Arbeitspunkt und Lokalisator ist z.B. durch die Baugröße des Lokalisators vorgegeben. Daher ist es mit Herkömmliche Lageerfassungssysteme nicht möglich, die Position des Arbeitspunkts des medizinischen Instruments bei nicht gebogenem Instrumentenschaft genau zu bestimmen.

Herkömmliche multiple-use Instrumente haben den Nachteil, dass vor jedem Einsatz der Arbeitspunkt im Lageerfassungssystem registriert werden muss, da das Instrument plastisch verformt sein kann. Bei single-use Instrumenten kommt noch das Problem dazu, dass die Lokalisatoren, die in dem single-use Instrument angeordnet sind, verhältnismäßig teuer sind und in der Regel gemeinsam mit dem single-use Instrument entsorgt werden müssen. Hierdurch entstehen regelmäßig hohe Zusatzkosten.

Daher ist es die Aufgabe der vorliegenden Erfindung, ein chirurgisches Instrument mit einer Lokalisatoranordnung bereitzustellen, mit dem die Position des Arbeitspunkts bei einer Biegung des Schafts deutlich genauer als bei herkömmlichen Lageerfassungssystemen bestimmbar ist. Ferner soll das chirurgische Instrument ohne zusätzlichen Registrierungsvorgang im Lageerfassungssystem verwendbar sein, und dennoch dem Lageerfassungssystem eine zuverlässige Bestimmung der Position des Arbeitspunkts ermöglichen. Schließlich soll eine single-use Ausführung des medizinischen Instruments geringere Zusatzkosten verursachen als ein herkömmliches single-use Instrument.

Erfindungsgemäß wird die Aufgabe durch ein chirurgisches Instrument mit einem Instrumentengriff, einem mit dem Instrumentengriff verbundenen Instrumentenschaft, der eine Instrumentenachse definiert, einer mit dem Instrumentenschaft verbundenen abgewinkelten Instrumentenspitze mit einem Arbeitspunkt, der nicht auf der Instrumentenachse angeordnet ist, sowie einem an dem Instrumentengriff oder dem Instrumentenschaft angeordneten ersten Lokalisator gelöst. Der erste Lokalisator erfasst sechs Freiheitsgrade. Weiterhin ist der erste Lokalisator als Lagesensor mit zweit Spulen ausgebildet. Der Schaft ist zwischen dem ersten Lokalisator und dem Arbeitspunkt während eines Einsatzes des chirurgischen Instruments, z.B. aufgrund einer auf den Arbeitspunkt des Instruments ausgeübten Kraft, auslenkbar. Ein zweiter Lokalisator ist mit Abstand zum ersten Lokalisator und gegenüber diesem näher an dem Arbeitspunkt des chirurgischen Instruments und in einem zur Instrumentenspitze benachbarten Bereich des Instrumentenschaftes angeordnet. Der zweite Lokalisator weist eine Spule auf und hat kleinere Abmessungen als der erste Lokalisator. Der zweite Lokalisator ist als Lagesensor ausgebildet nur fünf Freiheitsgrade zu erfassen. Auf diese Weise weist der zweite Lokalisator eine kleinere Baugröße als ein im Wesentlichen baugleicher Lokalisator, der zur Erfassung von sechs Freiheitsgraden ausgebildet ist. Ferner ist der zweite Lokalisator bei gleicher Bauart wie der erste Lokalisator kostengünstiger, da er weniger Komponenten benötigt. Da sich chirurgische Instrumente in der Regel vom Instrumentengriff zur Instrumentenspitze hin verjüngen und wegen der geringeren Abmessungen des zweiten Lokalisators, ist der zweite Lokalisator näher zum Arbeitspunkt am chirurgischen Instrument anordenbar als ein Lokalisator mit größerer Baugröße. Somit können Verbiegungen des chirurgischen Instruments zwischen dem ersten Lokalisator und dem zweiten Lokalisator ermittelt und bei der Bestimmung der Lage des Arbeitspunkts berücksichtigt werden. Hierfür kann auch eine Biegekennlinie des Instruments hinzugezogen werden, damit die Durchbiegung des Instruments zwischen dem zweiten Lokalisator und dem Arbeitspunkt zumindest rechnerisch ermittelt werden kann. Die Genauigkeit der Bestimmung des Arbeitspunkts ist somit gegenüber herkömmlichen Lageerfassungssystemen deutlich verbessert. Mit Hilfe des ersten Lokalisators kann auch der vom zweiten Lokalisator nicht erfasste Freiheitsgrad unter Zugrundelegung bekannter Geometriedaten bestimmt werden.

Bevorzugt ist der Arbeitspunkt das distale Ende der Instrumentenspitze.

Weil der erste Lokalisator sechs Freiheitsgrade erfasst, ist die Lage des chirurgischen Instruments von der Lageerfassungseinheit allein aufgrund der über den ersten Lagesensor ermittelten Daten bestimmbar. Der erste Lokalisator liefert dem Lageerfassungssystem die Lage des Instruments, während der zweite Lokalisator dem Lageerfassungssystem Informationen zur Bestimmung der Verformung des Instruments bereitstellt.

Der zweite Lokalisator weist kleinere Abmessungen auf als der erste Lokalisator. Da z.B. im Instrumentengriff ausreichend Platz für den ersten Lokalisator ist und sich das chirurgische Instrument von dem Instrumentengriff zum Arbeitspunkt hin verjüngt, kann der erste Lokalisator größere Abmessungen als der zweite Lokalisator haben. Je kleiner der zweite Lokalisator ist, desto näher ist er an dem Arbeitspunkt anordenbar.

Weiter bevorzugt weist der zweite Lokalisator einen Durchmesser von unter 0,5 mm, insbesondere unter 0,4 mm auf. Somit ist der zweite Lokalisator auch bei sich stark verjüngenden Instrumenten bzw. an relativ kleinen Durchmessern in der Nähe des Arbeitspunkts anordenbar.

Lagesensoren werden in elektromagnetischen Lageerfassungssystemen eingesetzt. Wird ein solcher Lagesensor in einem alternierenden elektromagnetischen Feld angeordnet, wird in dem Lagesensor in Abhängigkeit der Lage - also der Position und der Orientierung - des Lagesensors in den Spulen des Lagesensors ein Strom induziert. Aufgrund der bekannten Anordnung der Spulen und der Stärken der in den Spulen induzierten Ströme kann das Lageerfassungssystem die Lage des Lagesensors innerhalb des elektromagnetischen Felds bestimmen. Mit einer Spule sind fünf Freiheitsgrade bestimmbar. Durch dieses Merkmal kann der zweite Lokalisator eine einzige Spule aufweisen und bei entsprechender Bauweise im Inneren des chirurgischen Instruments angeordnet werden und ist somit vor äußeren Einflüssen geschützt. Dies ist insbesondere dann von Vorteil, wenn das chirurgische Instrument leicht sterilisierbar sein soll.

Der erste Lokalisator ist als Lagesensor ausgebildet und weist zwei Spulen auf. Dies hat die gleichen Vorteile wie beim zweiten Lagesensor. Für den Fall, dass der erste und der zweite Lokalisator als Lagesensor ausgebildet sind, ist ein rein elektromagnetisches Lageerfassungssystem ausreichend, um die Lage des chirurgischen Instruments sowie die Position des Arbeitspunkts im Referenzkoordinatensystem zu bestimmen.

Optional zusätzlich kann der erste Lokalisator mindestens einen Reflektor für Licht- und/oder Schallwellen aufweisen. Somit ist die Lage des chirurgischen Instruments über optische und/oder akustische Mittel bestimmbar. Derartige chirurgische Instrumente sind in z.B. Lageerfassungssystemen einsetzbar, die keinen Feldgenerator zum Emittieren eines alternierenden elektromagnetischen Felds aufweisen. Demnach ist nur noch für den zweiten Lokalisator ein elektromagnetisches Lageerfassungssystem erforderlich, das z.B. sehr kompakt ausgebildet sein kann und in ein bestehendes optisches und/oder akustisches Lageerfassungssystem leicht integrierbar ist.

Vorzugsweise weist der Instrumentenschaft eine größere Biegsamkeit als die Instrumentenspitze auf. Da das chirurgische Instrument in der Regel aus einem Material gefertigt ist, sind unterschiedliche Biegsamkeiten über unterschiedliche Flächenträgheitsmomente erzielbar. Eine größere Biegsamkeit wird demnach z.B. durch Verringerung des Querschnitts erzielt. Eine weitere Maßnahme zur Vergrößerung der Biegsamkeit ist ein Verändern der Form des Querschnitts, so dass das Instrument in einem solchen Abschnitt unterschiedliche, richtungsabhängige Biegsamkeiten aufweist.

Ebenfalls bevorzugt weist der Instrumentenschaft eine größere Biegsamkeit als die Instrumentenspitze auf. Hierdurch beschränkt sich die Verformung des chirurgischen Instruments im Wesentlichen auf den Instrumentenschaft, während die Instrumentenspitze einer geringeren bzw. vernachlässigbaren Verformung unterliegt. Bei bekannter Verformung des Instrumentenschafts, die leicht über den ersten und zweiten Lokalisatoren ermittelbar ist, ist somit die Position des Arbeitspunkts des Instruments leicht bestimmbar.

Weiter bevorzugt weist die Instrumentenspitze zwischen dem zweiten Lokalisator und dem Arbeitspunkt eine geringere Biegsamkeit als der übrige Teil der Instrumentenspitze auf. Dies bewirkt, dass die Position des Arbeitspunkts genauer bestimmbar ist, da sämtliche Verformungen bzw. Durchbiegungen des Instruments, die zwischen den Lokalisatoren erfolgen, über die zwei Lokalisatoren ermittelbar sind und eine etwaige Durchbiegung des Bereichs der Instrumentenspitze, der zwischen dem zweiten Lokalisator und dem Arbeitspunkt angeordnet ist, wegen der geringeren Biegsamkeit vernachlässigbar klein ist. Auch in diesem Fall ist es von Vorteil, wenn der zweite Lokalisator möglichst nach an dem Arbeitspunkt angeordnet ist, um die Genauigkeit der Bestimmung des Arbeitspunkts weiter zu erhöhen.

Vorzugsweise ist das chirurgische Instrument ein Sichelmesser oder ein Antrumlöffel.

In einer vorteilhaften Ausgestaltung der Erfindung weist das chirurgische Instrument eine Speichereinheit zum Speichern von Relativpositionsdaten des zweiten Lokalisators relativ zum ersten Lokalisator und/oder zum Speichern von Relativpositionsdaten des Arbeitspunkts relativ zum zweiten Lokalisator und/oder zum Speichern von Relativpositionsdaten des Arbeitspunkts relativ zum ersten Lokalisator auf. Somit können etwaige Verformungen des Instruments, die in einem Registrierungsprozess ermittelt wurden, im Instrument abgespeichert werden. Beim erneuten Registrieren des Instruments sind diese Daten vom Lageerfassungssystem abrufbar und eine erneute Registrierung nicht erforderlich. Ferner können somit auch bei unbenutzten Instrumenten bereits bei Auslieferung die Relativposition des Arbeitspunkts zum ersten Lokalisator bzw. zweiten Lokalisator abgespeichert sein, so dass eine Registrierung des Instruments im Lageerfassungssystem - sofern der Arbeitspunkt des Instruments nicht durch plastische Deformation verschoben wurde - nicht erforderlich ist.

Besonders bevorzugt ist der zweite Lokalisator innerhalb einer Außenkontur des chirurgischen Instruments angeordnet. Somit ist der zweite Lokalisator vor äußeren Einflüssen besser geschützt. Dies ist insbesondere von Vorteil, da das chirurgische Instrument leicht sterilisierbar sein muss. Ferner ist das Instrument leichter sterilisierbar, wenn die Oberfläche weniger Unebenheiten aufweist.

In einer bevorzugten Ausführungsform der Erfindung ist der zweite Lokalisator weniger als 2 cm vom Arbeitspunkt entfernt in dem chirurgischen Instrument angeordnet. Besonders bevorzugt ist der zweite Lokalisator weniger als 1 cm vom Arbeitspunkt entfernt in dem Instrument angeordnet. Je näher der Lokalisator am Arbeitspunkt angeordnet ist, desto genauer ist die Position des Arbeitspunkts vom Lageerfassungssystem bestimmbar, da eine etwaige Durchbiegung des Instruments über einen größeren Teil des Instruments messbar und somit exakter ermittelbar ist.

Vorzugsweise sind der erste Lokalisator und der zweite Lokalisator derart an dem chirurgischen Instrument angeordnet, dass das Lageerfassungssystem die Art des verwendeten chirurgischen Instruments aufgrund dem Lageerfassungssystem bereitgestellter Kennwerte über chirurgische Instrumente und die jeweilige Anordnung der Lokalisatoren an dem chirurgischen Instrument erkennt. Demnach ist es bevorzugt, dass jedes chirurgische Instrument, das in einem Lageerfassungssystem verwendet wird, eine individuelle Anordnung der Lokalisatoren - relative Lage- zueinander aufweist. Beim Registrieren des chirurgischen Instruments im Lageerfassungssystem, bei dem das chirurgische Instrument nicht durch Druckkräfte verformt ist, erkennt das System automatisch die Lage der ersten Lokalisators und des zweiten Lokalisators und kann somit die relative Lage der Lokalisatoren zueinander bestimmen. Über einen Abgleich der relativen Lage der Lokalisatoren mit einer Datenbank, in der chirurgische Instrumente mit den jeweiligen relativen Lagen der Lokalisatoren abgespeichert sind, kann das Lageerfassungssystem das chirurgische Instrument sowie die relevanten Kennwerte, wie z.B. die Bezeichnung, die Abmessungen des Instruments oder die Lage des Arbeitspunkts zum ersten bzw. zweiten Lokalisator, bestimmen. Ein Einmessen des Arbeitspunkts im Lageerfassungssystem ist somit nur noch in Fällen notwendig, in denen das Instrument plastisch verformt ist.

Weiter bevorzugt sind die Instrumentenspitze mit dem Instrumentenschaft lösbar an dem Instrumentengriff anordenbar, wobei der erste Lokalisator in dem Instrumentengriff und der zweite Lokalisator in dem Instrumentenschaft angeordnet ist. Alternativ oder zusätzlich kann die Instrumentenspitze lösbar an dem Instrumentenschaft angeordnet sein. In dieser alternativen Ausführungsform ist der zweite Lokalisator in der Instrumentenspitze und der erste Lokalisator in dem Instrumentengriff oder dem Instrumentenschaft angeordnet. Diese Ausführungsform ist nicht Teil der Erfindung. Nach dem Zusammensetzen von Instrumentengriff, Instrumentenschaft und Instrumentenspitze sind der erste Lokalisator und der zweite Lokalisator und somit der Arbeitspunkt des chirurgischen Instruments vom Lageerfassungssystem erfassbar. Das Instrument ist somit automatisch kalibrierbar. Ferner muss bei einem single-use Instrument nach dem Einsatz nur der Teil des chirurgischen Instruments entsorgt werden, der den kostengünstigeren zweiten Lokalisator Der zweite Lokalisator kann in diesem Fall beispielsweise konzentrisch um das Lumen herum in Form einer Hohlspule, innerhalb oder außerhalb des Lumens angeordnet sein. Weiter bevorzugt weisen der Instrumentenschaft und der Instrumentengriff ebenfalls ein durchgängiges Lumen auf, das mit dem Lumen von Instrumentenspitze ein durchgängiges Lumen aufweist, wenn Instrumentengriff, Instrumentenschaft und Instrumentenspitze zusammengefügt sind. Der Instrumentengriff hat an einem proximalen Ende eine Öffnung zu dem Lumen. Die Öffnung kann z.B. als Anschluss für einen Schlauch ausgebildet sein.

Vorzugsweise ist die Instrumentenspitze über eine Klemm- oder Luer-Lock-Verbindung mit dem Instrumentenschaft bzw. der Instrumentenschaft mit dem Instrumentengriff verbindbar. Eine Klemmverbindung kann beispielsweise eine Ausnehmung in dem Instrumentenschaft bzw. Instrumentengriff umfassen, in die die Instrumentenspitze bzw. der Instrumentenschaft einsetzbar und mittels einer Klemmschraube in dieser Ausnehmung festsetzbar ist. Im Falle einer Luer-Lock-Verbindung ist es vorteilhaft, wenn die Instrumentenspitze bzw. der Instrumentenschaft den männlichen Teil und der Instrumentenschaft bzw. der Instrumentengriff den weiblichen Teil der Luer-Lock-Verbindung aufweist. Über derartige Verbindungen sind Instrumentengriff, Instrumentenschaft und Instrumentenspitze sicher und wieder lösbar aneinander gehalten, so dass eine ungewollte Relativbewegung zwischen miteinander verbundenen Instrumentengriff, Instrumentenschaft und Instrumentenspitze verhindert wird. Erfindungsgemäß kann aber auch eine nahezu beliebig ausgestaltete Steck- und/oder Schraubverbindung vorgesehen sein.

Vorteilhaft weisen Instrumentengriff, Instrumentenschaft und Instrumentenspitze elektrische Anschlüsse auf, über die beim Koppeln von Instrumentengriff, Instrumentenschaft und Instrumentenspitze eine elektrische Verbindung zwischen den Instrumententeilen herstellbar ist. Eine solche elektrische Verbindung kann zum Übermitteln von elektrischen Signalen sowie zur Bereitstellung einer elektrischen Spannung eingerichtet sein. Vorzugsweise umfasst die elektrische Verbindung einen Stecker sowie eine passende Buchse, wobei die Pole der elektrischen Verbindung vorzugsweise koaxial angeordnet sind. In einer vorteilhaften Ausgestaltung der Erfindung umfasst die elektrische Verbindung einen koaxial mit der Instrumentenachse anordenbaren, mehrpoligen Klinkenstecker sowie eine dazu passende Klinkenbuchse. Erfindungsgemäß kann die elektrische Verbindung auch elektrisch leitende Kontaktplatten umfassen, an die elektrisch leitende Kontaktelemente unter Beaufschlagung einer Federkraft pressbar sind. Die Kontaktelemente können beispielsweise gebogenen Blechstreifen umfassen, die aufgrund ihrer Form eine Federkraft erzeugen können. Alternativ können die Kontaktelemente Kugeln umfassen, die beispielsweise von einer Spiralfeder in Richtung der entsprechenden Kontaktplatte gedrückt werden.

Bevorzugt weist die Instrumentenspitze und/oder der Instrumentenschaft eine Identifikationseinrichtung auf, die Informationen über die Instrumentenspitze bereitstellt. Weiter bevorzugt ist die Identifikationseinrichtung ausgebildet, die Informationen über die Identität der Instrumentenspitze einer Steuereinheit des medizinischen Instruments bereitzustellen. Die Steuereinheit ist bevorzugt außerhalb des chirurgischen Instruments angeordnet, beispielsweise in einem von dem chirurgischen Instrument separaten Gehäuse. Über Signalübertragungsmittel, wie z.B. Kabel oder Funkwellen ist die Steuereinheit mit dem chirurgischen Instrument koppelbar.

Die Identifikationseinrichtung kann Informationen über die Eigenschaften der Instrumentenspitze umfassen. Hierzu können beispielsweise Lage und Ausrichtung des Arbeitspunkts der Instrumentenspitze, insbesondere relativ zu dem Bereich der Instrumentenspitze, der im Betrieb mit dem Instrumentenschaft in Eingriff kommt, Form, Material, Härte sowie Flexibilität und/oder mechanische Belastbarkeit der Instrumentenspitze zählen. Alternativ können diese oder ein Teil dieser Daten in einer mit der Steuereinheit gekoppelten Speichereinheit abgespeichert sein, so dass die Steuereinheit nach der Identifizierung einer auf den Instrumentenschaft aufgesetzten Instrumentenspitze die zu der jeweiligen Instrumentenspitze zugehörigen Daten aus der Speichereinheit auslesen kann. Ferner umfasst die Identifikationseinrichtung vorzugsweise entsprechende Informationen über den Instrumentenschaft.

Vorzugsweise weist die Identifikationseinrichtung Informationen über Typ, Bauart und/oder Modellbezeichnung der Instrumentenspitze auf. Derartige Informationen dienen der Steuereinheit der genauen Identifikation der verwendeten Instrumentenspitze. Diese Information sind mit gespeicherten Informationen über die Instrumentenspitze vergleichbar, so dass auf Basis dieser Informationen beispielsweise Lage und Ausrichtung des Arbeitspunkts der Instrumentenspitze, insbesondere relativ zu dem Bereich der Instrumentenspitze, der ggf. mit dem Instrumentenschaft in Eingriff kommt, Form, Material, Härte sowie Flexibilität und/oder mechanische Belastbarkeit der Instrumentenspitze ermittelbar sind. Ebenso können die Informationen genutzt werden, um bestimmte Eigenschaften der Software zu steuern (z.B. Visualisierung des Navigationspunktes, Visualisierung der Geometrie des Instruments, welche Schnittbilder werden angezeigt, Zielansicht ggf einblenden, Filter für die Signaldatenverarbeitung wählen). Ferner umfasst die Identifikationseinrichtung vorzugsweise entsprechende Informationen über den Instrumentenschaft.

Gemäß einer bevorzugten Ausführungsform sind die Informationen der Identifikationseinrichtung über den Instrumentenschaft an die Steuereinheit übermittelbar. Hierfür kann der Instrumentenschaft oder der Instrumentengriff Auslesemittel wie z.B. einen Scanner oder Oberflächenabtaster aufweisen. Ferner kann eine elektrische Verbindung zwischen Instrumentenspitze und Instrumentenschaft zum Übermitteln der Informationen vorgesehen sein. Alternativ kann das Auslesemittel in einer Ausleseeinheit angeordnet sein, die außerhalb des medizinischen Instruments angeordnet und mit der Steuereinheit zu verbinden ist, z.B. über ein Kabel zur Datenübermittlung oder kabellos über eine Funkverbindung.

Bevorzugt weist die Identifikationseinrichtung einen elektronischen Speicher, einen graphisch dargestellten Code, einen Magnetcode und/oder eine Steckverbindung auf. In dem elektronischen Speicher, wie z.B. einem ROM oder EPROM, sind die entsprechenden Informationen als elektrische Signale gespeichert und können auf bekannte Weise ausgelesen werden. Ein graphisch dargestellter Code könnte beispielsweise ein Barcode sein, der über einen Laserscanner eingelesen werden kann. Ferner können Informationen auf Magnetisierten Bereichen in Form eines Magnetcodes abgelegt sein. Als Steckverbindung können beispielsweise Brückenschaltungen in Betracht kommen, bei denen ein bestimmter Code durch die Verbindung entsprechender Kontakte bzw. Pins mittels geeigneter elektrisch leitender Brücken darstellbar ist.

In einer bevorzugten Ausführungsform der Erfindung weist die Instrumentenspitze und der Instrumentenschaft mindestens ein durchgängiges Lumen auf, z.B. bestimmt als Spül- Saugröhrchen.

In einer alternativen Ausführungsform der Erfindung ist der zweite Lokalisator oder der erste Lokalisator in einer Bohrhülse angeordnet. Die Bohrhülse ist am Patienten anordenbar und das chirurgische Instrument ist bestimmungsgemäß in die Bohrhülse einführbar. Somit ist die Lage der Bohrhülse relativ zum Patienten bestimmbar. Da das chirurgische Instrument in die Bohrhülse einführbar ist, kann über die Einführtiefe des Instruments in der Bohrhülse die Position des Arbeitspunkts des Instruments ermittelt werden. Dies ist möglich, da das Instrument nur entlang einer Bahn relativ zur Bohrhülse verschiebbar ist. Die Tiefe kann somit z.B. durch Messmarkierungen an dem chirurgischen Instrument ermittelt werden. Alternativ kann das chirurgische Instrument ebenfalls einen Lokalisator zur Bestimmung der Tiefe und des Arbeitspunkts aufweisen. Über die Bestimmung der Lage der Bohrhülse zum Patienten sowie der Lage des chirurgischen Instruments relativ zur Bohrhülse ist die Lage des chirurgischen Instruments relativ zum Patienten ermittelbar. Diese Ausführungsform hat den Vorteil, dass auch der Arbeitspunkt eines chirurgischen Instruments, das nicht an ein Lageerfassungssystem angebunden ist, über die Lage der Bohrhülse zum Patienten sowie die Lage des chirurgischen Instruments zur Bohrhülse bestimmbar ist.

Ferner wird die Aufgabe der Erfindung durch ein Lageerfassungssystem zur Ermittlung der Lage von chirurgischen Instrumenten, mit einer Auswerteeinheit zum Ermitteln der Lage von in dem Lageerfassungssystem angeordneten Lokalisatoren und einer Anzeigeeinheit zur lagegetreuen Darstellung des chirurgischen Instruments gelöst, wobei das chirurgische Instrument eine erfindungsgemäße Lokalisatoranordnung aufweist.

Weiterhin ist ein Verfahren zur Lageerfassung eines chirurgischen Instruments offenbart, wobei das Instrument einen Instrumentengriff, einen mit dem Instrumentengriff verbundenen und während eines Einsatzes des chirurgischen Instruments auslenkbaren Instrumentenschaft, eine mit dem Instrumentenschaft verbundene Instrumentenspitze und mit einem Arbeitspunkt aufweist, und wobei dem Instrument ein erster Lokalisator zumindest in einem Zeitpunkt räumlich definiert zugeordnet ist, und ein zweiter Lokalisator am Instrument nahe der Instrumentenspitze angeordnet ist, wobei der zweite Lokalisator ausgebildet ist, fünf Freiheitsgrade zu erfassen, wobei das Verfahren die Schritte aufweist:
- Bestimmen eines instrumentenspezifischen Kalibrierungsvektors vom zweiten Lokalisator zum Arbeitspunkt im Koordinatensystem des ersten Lokalisators bei unausgelenktem Instrumentenschaft , wobei die Bestimmung bevorzugt initial erfolgt;
- Bestimmen der Position und Richtung des Arbeitspunktes im Referenzkoordinatensystem durch Transformation einer um den Kalibrierungsvektor verschobenen Momentankoordinate des zweiten Lokalisators in das Koordinatensystem des ersten Lokalisators, wobei die Transformation bevorzugt in jedem Messzyklus erfolgt.

Bevorzugt ist vorgesehen, dass der zweite Lokalisator eine Position und der erste Lokalisator eine Richtung für die Transformation des Arbeitspunktes liefert.

In einer bevorzugten Ausgestaltung des Verfahrens ist der erste Lokalisator an dem Instrumentengriff oder dem Instrumentenschaft angeordnet und der Instrumentenschaft ist zwischen dem ersten Lokalisator und dem Arbeitspunkt während eines Einsatzes des chirurgischen Instruments auslenkbar. Der der zweite Lokalisator kann mit Abstand zum ersten Lokalisator und gegenüber diesem näher an dem Arbeitspunkt angeordnet sein,

In einer besonders bevorzugten Ausgestaltung liegt der Arbeitspunkt außerhalb einer Instrumentenachse liegt.

Der erste Lokalisator kann ein Patentenlokalisator sein, der getrennt vom Instrument und patientenfest angeordnet ist. Der erste Lokalisator kann eine Bohrhülse sein, die getrennt vom Instrument und patientenfest angeordnet ist. Bevorzugt ist das Instrument in die Bohrhülse einführbar und das Verfahren weist den Schritt auf:
- Ermitteln einer Position des Arbeitspunkts des Instruments über eine Einführtiefe des Instruments in die Bohrhülse.

Es hat sich als vorteilhaft herausgestellt, wenn das Verfahren ein Speichern des Kalibrierungsvektors in einem Speicher, insbesondere in einem Instrumentenspeicher und ein Bestimmen der Position und Richtung des Arbeitspunktes im Referenzkoordinatensystem nach Auslesen des Kalibrierungsvektors aus dem Speicher aufweist.

Die Erfindung schließt die Erkenntnis ein, dass chirurgische Instrumente für minimalinvasive Zugang oder Zugänge durch natürliche Körperöffnungen (z.B. Nase) mit dünnen Schäften ausgestattet sind (Antrumlöffel, Sichelmesser etc.). Bei diesen Instrumenten kann der Arbeitspunkt (Instrumentenspitze) außerhalb der Achse des Schaftes liegen, wodurch beim Arbeiten mit dem Instrument mit einer elastischen Verformung des Schaftes durch die anliegenden Kräfte zu rechnen ist.

Um solche chirurgischen Instrumente mit einem dünnen Schaft zu navigieren, kann ein Lokalisator in den Schaft integriert sein, bevorzugt unmittelbar am Ende des Arbeitspunktes. Dadurch ist die Verformung des Schaftes für die Positionserfassung des Arbeitspunktes vernachlässigbar.

Die Erfindung schließt ebenfalls die Erkenntnis ein, dass der Lokalisator mit einem geringen Durchmesser ausgeführt werden (<0,5 mm, insbesondere <0.4 mm) kann, wenn auf einen Freiheitsgrad verzichtet wird (5DOF Sensor).

Fehler der Orientierungsinformation (im 6DOF Sensor) wirken sich geringer aus als in Instrumenten mit Sensoren nur im oder am Griffstück, da die Strecke zwischen 5DOF Sensor und navigiertem Instrumentenarbeitspunkt gering ist.

Um einen Instrumentenarbeitspunkt außerhalb der Spulen-, bzw. Schaftachse zu kalibrieren, kann ein zweiter und dritter Spulensensor im Instrument (z. B. Griff) integriert sein. Diese Sensoren bilden zusammen einen Lokalisator mit 6 Freiheitsgraden (6 DOF Sensor).

Ferner ist ein Verfahren zur Kalibrierung eines ersten Lokalisators eines Instrumentenschafts auf einen Arbeitspunkt einer an dem Instrumentenschaft in einer Arbeitsposition anordenbaren Instrumentenspitze in einem Lageerfassungssystem mit einer Steuereinheit offenbart, wobei die Instrumentenspitze einen zweiten Lokalisator aufweist, der auf den Arbeitspunkt der Instrumentenspitze kalibriert ist. Das Verfahren ist gekennzeichnet durch die Schritte:
- Anordnen der Instrumentenspitze an dem Instrumentenschaft in der Arbeitsposition,
- Registrieren der Instrumentenspitze an der Steuereinheit,
- Ermitteln der Geometrie der Instrumentenspitze sowie der Lage des zweiten Lokalisators zum ersten Lokalisator und dem Arbeitspunkt auf Basis der bei der Registrierung der Instrumentenspitze gesammelten Informationen über die Instrumentenspitze,
- Erfassen der Lage des Arbeitspunktes der Instrumentenspitze innerhalb des Lageerfassungssystems aufgrund des Sensorsignals des zweiten Lokalisators sowie der ermittelten Informationen über die Geometrie der Instrumentenspitze,
- Erfassen des Sensorsignals des ersten Lokalisators
- Kalibrieren des zweiten Lokalisators auf den Arbeitspunkt der Instrumentenspitze unter Verwendung der Sensorsignale des zweiten Lokalisators und des ersten Lokalisators sowie der Information über die Lage des Arbeitspunkts relativ zum zweiten Lokalisator.

Die Instrumentenspitze kann sowohl vor als auch nach bzw. während dem Anordnen der Instrumentenspitze an dem Instrumentenschaft in der Arbeitsposition an der Steuereinheit registriert werden. Die Daten über die Geometrie der Instrumentenspitze und/oder der Lage des zweiten Lokalisators zum Arbeitspunkt der Instrumentenspitze können entweder in einer Speichereinheit in der Instrumentenspitze oder einer außerhalb der Instrumentenspitze angeordneten Datenbank gespeichert sein. Im letzteren Fall ist es ausreichend, dass beim Registrieren der Instrumentenspitze an der Steuereinheit die Instrumentenspitze lediglich identifiziert wird und die Steuereinheit die entsprechenden Daten der jeweiligen Instrumentenspitze aus einer Speichereinheit ausliest.

Der erste Lokalisator kann ein proximaler Sensors eines Instrumentenschafts sein. Der zweite Lokalisator kann ein. Der zweite Lokalisator kann ein distaler Sensor einer Instrumentenspitze sein.

Im Folgenden soll die Erfindung anhand eines Ausführungsbeispiels unter Bezugnahme auf Figuren näher erläutert werden. In den Figuren zeigt:
- Fig. 1:: eine schematische Seitenansicht einer einteiligen Ausführungsform eines erfindungsgemäßen chirurgischen Instruments;
- Fig. 2:: eine schematische Seitenansicht einer Ausführungsform eines chirurgischen Instruments, welches nicht Teil der Erfindung ist;
- Fig. 3:: eine schematische Ansicht eines erfindungsgemäßen Lageerfassungssystems zur Ermittlung der Lage von chirurgischen Instrumenten; und
- Fig. 4:: eine schematische Seitenansicht eines chirurgischen Instruments mit optischen Reflektoren als erstem Lokalisator für eine optische Lageerfassung.
- Fig. 5:: eine schematische Darstellung eines Verfahrens zur Lageerfassung eines chirurgischen Instruments.

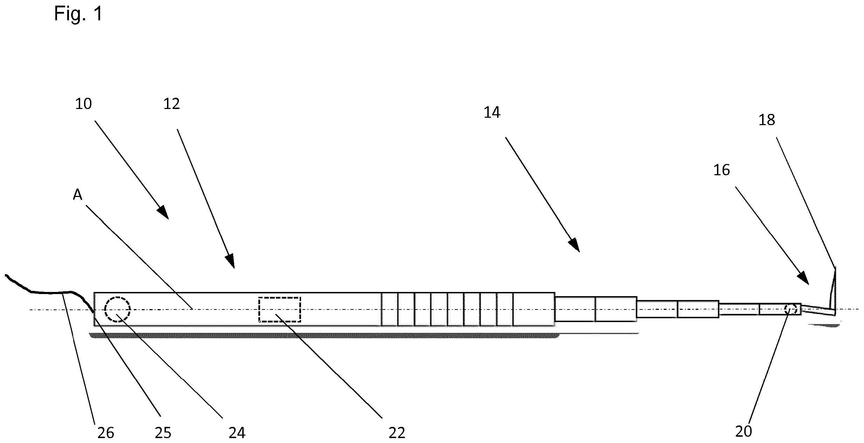

Das in Fig. 1 erfindungsgemäße gezeigte chirurgische Instrument 10 weist eine Instrumentenachse A, einen länglichen, im Wesentlichen zylindrischen Instrumentengriff 12, einen an einem distalen Ende des Instrumentengriffs 12 koaxial angeordneten, länglichen, zylinderförmigen Instrumentenschaft 14 und eine an einem distalen Ende des Instrumentenschafts 14 angeordnete, abgewinkelte Instrumentenspitze 16 auf. Anstatt zylinderförmig kann das chirurgische Instrument 10 auch im Wesentlichen quaderförmig oder mit ovalem Querschnitt ausgebildet sein. Die Instrumentenspitze 16 weist an einem distalen Ende einen Arbeitspunkt 18 auf, der in dieser Ausführungsform nicht auf der Instrumentenachse A angeordnet ist. In alternativen Ausführungsformen kann der Arbeitspunkt 18 auch auf der Instrumentenachse A angeordnet sein.

Der Instrumentenschaft 14 weist einen kleineren Durchmesser als der Instrumentengriff 12 und die Instrumentenspitze 16 einen kleineren Durchmesser als der Instrumentenschaft 14 auf. Der Instrumentenschaft 14 ist in dieser Ausführungsform um ein Vielfaches länger als die Instrumentenspitze. Eine Kraftkomponente, die senkrecht zur Instrumentenachse auf den Arbeitspunkt 18 wirkt, verursacht eine Verbiegung des chirurgischen Instruments 10 im Wesentlichen im Bereich des Instrumentenschafts 14.

In einem der Instrumentenspitze 16 benachbarten Bereich des Instrumentenschafts 14 ist ein zweiter Lokalisator 20 angeordnet. Der zweite Lokalisator 20 ist vorzugsweise in einer Kavität des Instrumentenschafts 14 angeordnet. Ein in dieser Darstellung nicht erkennbares Lokalisatorkabel verläuft im Inneren des chirurgischen Instruments 10 vom zweiten Lokalisator 20 zu einem proximalen Ende 25 des chirurgischen Instruments 10. Der zweite Lokalisator 20 ist möglichst nah an dem Arbeitspunkt 18 der Instrumentenspitze 16 angeordnet. Der Lokalisator 20 kann auch in der Instrumentenspitze 16 angeordnet sein. Vorzugsweise ist der zweite Lokalisator 20 ein Lagesensor mit einer einzigen Spule für ein elektromagnetisches Lageerfassungssystem.

In einer alternativen, nicht dargestellten Ausführungsform der Erfindung weist das chirurgische Instrument 10 mehrere zweite Lokalisatoren 20 auf, die entlang der Instrumentenachse A nebeneinander in dem Instrumentenschaft 14 angeordnet sind. Diese Anordnung ermöglicht eine Reduktion der von den einzelnen zweiten Lokalisatoren 20 ermittelbaren Freiheitsgrade und somit eine Reduktion der Baugröße der einzelnen zweiten Lokalisatoren 20. Dabei ist es wichtig, dass diese Vielzahl an zweiten Lokalisatoren 20 in ihrer Gesamtheit dazu ausgebildet ist, die gleiche Anzahl an Freiheitsgraden wie ein einzelner zweiter Lokalisator 20 zu ermitteln.

In der in Fig. 1 gezeigten, bevorzugten Ausführungsform der Erfindung ist ein erster Lokalisator 24 in der Nähe des proximalen Endes 25 in dem Instrumentengriff 12 angeordnet. Der erste Lokalisator 24 ist vorzugsweise in einer Kavität des Instrumentengriffs 12 angeordnet. Ein nicht dargestelltes Lokalisatorkabel führt vom ersten Lokalisator 24 zum proximalen Ende 25 des chirurgischen Instruments 10 und verläuft vorzugsweise im Inneren des Instrumentengriffs 12. In einer alternativen Ausführungsform ist der erste Lokalisator 24 in einem anderen Bereich des Instrumentengriffs 12 oder in dem Instrumentenschaft 14 angeordnet, wobei der erste Lokalisator 24 näher an dem proximalen Ende 25 des chirurgischen Instruments 10 als der zweite Lokalisator 20 angeordnet ist. Vorzugsweise ist der erste Lokalisator 24 ein Lagesensor mit zwei Spulen zur Verwendung in einem elektromagnetischen Lageerfassungssystem, so dass der erste Lokalisator 24 alle sechs Freiheitsgrade erfassen kann.

In einer alternativen, nicht dargestellten Ausführungsform kann das chirurgische Instrument 10 mehrere erste Lokalisatoren 24 aufweisen, die in dem Instrumentengriff 12 und/oder dem Instrumentenschaft 14 angeordnet sind. Die ersten Lokalisatoren 24 sind vorzugsweise dazu ausgebildet, in ihrer Gesamtheit mindestens sechs Freiheitsgrade zu erfassen. Vorzugsweise erfassen die ersten Lokalisatoren 24 redundante Signale, so dass z.B. der Defekt eines ersten Lokalisators 24 leicht ermittelbar ist.

In der in Fig. 1 gezeigten, bevorzugten Ausführungsform der Erfindung ist eine Speichereinheit 22 im Instrumentengriff 12 angeordnet. Von der Speichereinheit geht ein nicht dargestelltes Speicherkabel zum proximalen Ende 25 des chirurgischen Instruments 10 und verläuft vorzugsweise im Inneren des Instrumentengriffs 12. In der Speichereinheit 22 sind Lagedaten z.B. relative Lagen vom Arbeitspunkt 18 zum ersten Lokalisator 24 und/oder zum zweiten Lokalisator 20 und/oder vom ersten Lokalisator 24 zum zweiten Lokalisator 20 abgespeichert bzw. abspeicherbar. Diese Lagedaten sind vom Lageerfassungssystem auslesbar und ein chirurgisches Instrument 10 somit im Lageerfassungssystem registrierbar.

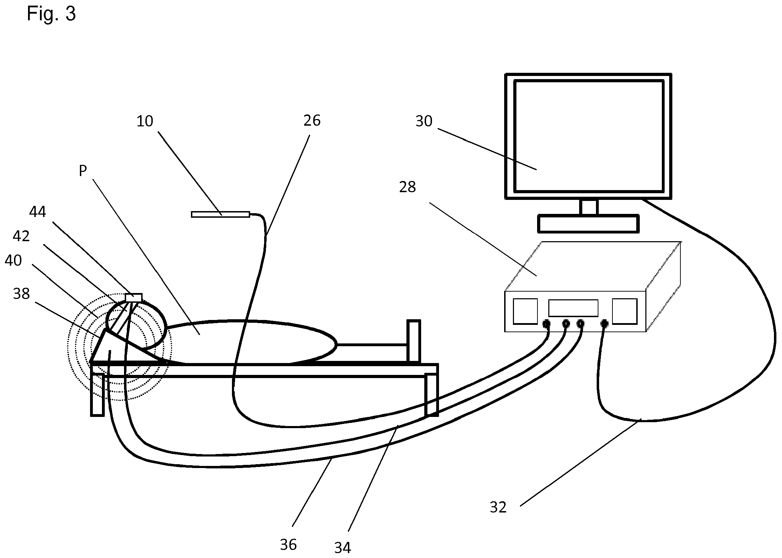

Die Lokalisatorkabel und das Speicherkabel verlaufen in einem Instrumentenkabel 26 vom proximalen Ende 25 des chirurgischen Instruments 10 zu einer in Fig. 3 dargestellten Auswerteeinheit 28.

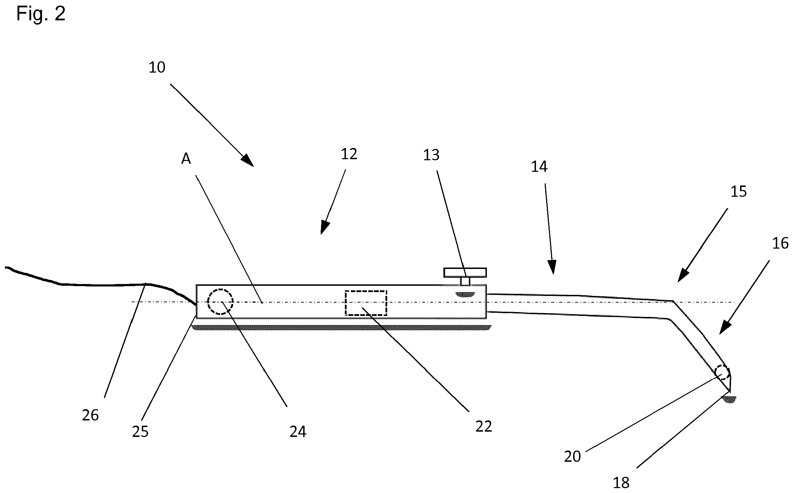

Das in Fig. 2 schematisch dargestellte, zweiteilige chirurgische Instrument 10 weist einen Instrumentengriff 12, einen Instrumentenschaft 14 und eine Instrumentenspitze 16 mit einem an einem distalen Ende der Instrumentenspitze 16 angeordneten Arbeitspunkt 18 auf. In der Instrumentenspitze 16 ist ein zweiter Lokalisator 20 dem Arbeitspunkt 18 unmittelbar benachbart angeordnet. Die Position des zweiten Lokalisators 20 entspricht somit im Wesentlichen der Position des Arbeitspunkts 18. Ferner ist ein Übergangsbereich zwischen der Instrumentenspitze 16 und dem Instrumentenschaft 14 abgekröpft ausgebildet. In alternativen, nicht dargestellten Ausführungsformen können Instrumentenschaft 14, Instrumentenspitze 16 sowie der Übergangsbereich 15 nahezu jede für ein chirurgisches Instrument geeignete Form aufweisen.

In dem in Fig. 2 dargestellten Instrument ist dem proximalen Ende 25 benachbart ein erster Lokalisator 24 im Instrumentengriff 12 angeordnet. Alternativ kann der erste Lokalisator 24 auch in einem beliebigen anderen Bereich des Instrumentengriffs 12 oder Instrumentenschafts 14 angeordnet sein.

Ein proximales Ende des Instrumentenschafts 14 ist in dem distalen Ende des Instrumentengriffs 12 aufgenommen und über eine Klemmschraube 13 in dieser Lage fixiert. In dem dargestellten Ausführungsbeispiel weisen die miteinander im Eingriff stehenden Bereiche von Instrumentenschaft 14 und Instrumentengriff 12 eine runde Querschnittsfläche auf. Bevorzugt sind diese Teilabschnitte abgeflacht oder weisen einen vieleckigen, insbesondere dreieckigen, quadratischen, fünfeckigen oder sternförmigen Querschnitt und/oder eine Führungsbahn z.B. nach dem Absatz-Nut-Prinzip auf, um ein relatives Verdrehen von Instrumentenschaft 14 und Instrumentengriff 12 durch Formschluss zu verhindern. Es sind Ausführungsformen vorgesehen, bei denen diese Teilabschnitte derart ausgebildet sind, dass Instrumentengriff 12 und Instrumentenschaft 14 nur in einer festgelegten gegenseitigen Ausrichtung miteinander in Eingriff bringbar sind.

In dem in Fig. 2 dargestellten zusammengesetzten Zustand des chirurgischen Instruments 10 sind Instrumentengriff 12 und Instrumentenschaft 14 sowohl mechanisch als auch elektrisch über in dieser Ansicht nicht sichtbare elektrische Kontakte miteinander verbunden. Über diese elektrische Verbindung sind Sensorsignale des zweiten Lokalisators 20 an den Instrumentengriff 12 übertragbar. An dem proximalen Ende 25 des Instrumentengriffs 12 ist ein Instrumentenkabel 26 zur Übermittlung der Lokalisatordaten des ersten Lokalisators 24 sowie des zweiten Lokalisators 20 an eine in Fig. 3 dargestellte Auswerteeinheit 28 angeordnet.

Das in Fig. 3 dargestellte Beispiel eines elektromagnetischen Lageerfassungssystems weist ein erfindungsgemäßes chirurgisches Instrument 10 auf, das über das Instrumentenkabel 26 mit der Auswerteeinheit 28 verbunden ist. Die Auswerteeinheit 28 ist über ein Anzeigekabel 32 mit einer Anzeigeeinheit 30 verbunden, um z.B. eine Abbildung des chirurgischen Instruments 10 lagegetreu in einem Referenzkoordinatensystem darzustellen. Wahlweise sind präoperativ und/oder intraoperativ gewonnene Bilddaten eines Patienten P überlagert und/oder nebeneinander bzw. übereinander auf der Anzeigeeinheit 30 darstellbar. Vorzugsweise ist die Anzeigeeinheit 30 ausgebildet, den Arbeitspunkt 18 des chirurgischen Instruments 10 und/oder Strukturen des Patienten P, die für den Operateur von besonderem Interesse sind, wie z.B. sensible Strukturen, die nicht beschädigt werden sollen oder Gewebe, das behandelt oder entfernt werden muss, graphisch besonders hervorzuheben, z.B. durch farbliches Markieren oder entsprechende Hinweiszeichen.

Die Auswerteeinheit 28 ist über ein Patientenlokalisatorkabel 34 mit einem Patientenlokalisator 44 verbunden, der über ein Befestigungsband 42 an der Stirn des Patienten P gehalten ist. Mittels des Patientenlokalisators 44 ist der Patient P in dem Lageerfassungssystem registrierbar, um die Lage des Patienten P im Referenzkoordinatensystem zu ermitteln. Ein Feldgeneratorkabel 36 verbindet die Auswerteeinheit 28 mit einem Feldgenerator 38, der in diesem Beispiel als Kopfstütze ausgebildet ist. Der Feldgenerator 38 emittiert ein alternierendes elektromagnetisches Feld 40, welches sich zumindest über den Bereich des Patienten P erstreckt, in dem die Operation durchzuführen ist. Das in Fig. 2 dargestellte Lageerfassungssystem ist somit in der Lage, die Position des Arbeitspunkts 18 des chirurgischen Instruments 10 genau zu bestimmen, auch wenn der Arbeitspunkt 18 des chirurgischen Instruments 10 aus seiner relativen Lage zum Instrumentengriff 12 bzw. zum ersten Lokalisator 24, z.B. aufgrund während der Operation auf das chirurgische Instrument 10 ausgeübter Druckkräfte, ausgelenkt wird.

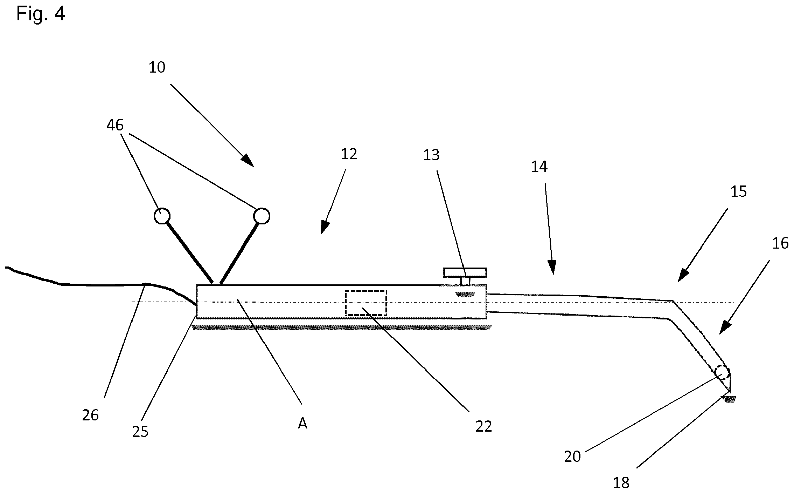

Figur 4 zeigt schematisch, dass anstelle eines oder zusätzlich zu einem ersten Lokalisator 24 in Form eines Lagesensors mit Sensorspulen auch ein optischer Lokalisator mit mehreren Reflektoren 46 vorgesehen sein kann.

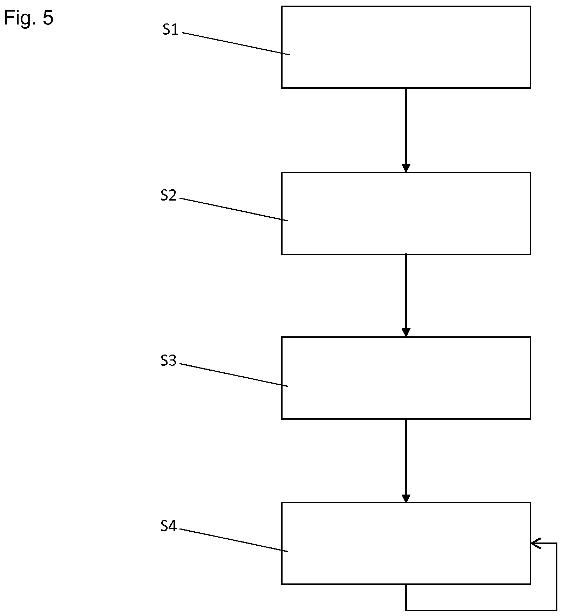

Eine schematische Darstellung eines Verfahrens zur Lageerfassung eines chirurgischen Instruments, beispielsweise des mit Bezug auf Fig. 1 beschriebenen Instruments, ist in Fig. 5 gezeigt.

In einem ersten Schritt S1 erfolgt initial ein Bestimmen eines instrumentenspezifischen Kalibrierungsvektors vom zweiten Lokalisator zum Arbeitspunkt im Koordinatensystem des ersten Lokalisators bei unausgelenktem Instrumentenschaft.

In einem zweiten Schritt S2 erfolgt ein Speichern des Kalibrierungsvektors in einem Instrumentenspeicher.

In einen dritten Schritt S3 wird der Kalibrierungsvektor aus dem Speicher ausgelesen.

In einem vierten Schritt S4 erfolgt ein Bestimmen der Position und Richtung des Arbeitspunktes im Referenzkoordinatensystem durch Transformation einer um den Kalibrierungsvektor verschobenen Momentankoordinate des zweiten Lokalisators in das Koordinatensystem des ersten Lokalisators.

Die Transformation erfolgt in jedem Messzyklus.

### Bezugszeichen:

- 10: Chirurgisches Instrument
- 12: Instrumentengriff
- 13: Klemmschraube
- 14: Instrumentenschaft
- 15: Übergangsbereich
- 16: Instrumentenspitze
- 18: Arbeitspunkt
- 20: Zweiter Lokalisator
- 22: Speichereinheit
- 24: Erster Lokalisator
- 25: Proximales Ende
- 26: Instrumentenkabel
- 28: Auswerteeinheit
- 30: Anzeigeeinheit
- 32: Anzeigekabel
- 34: Patientenlokalisatorkabel
- 36: Feldgeneratorkabel
- 38: Feldgenerator
- 40: Elektromagnetisches Feld
- 42: Befestigungsband
- 44: Patientenlokalisator
- 46: Optischer Reflektor
- A: Instrumentenachse
- P: Patient

## Patentansprüche

1. Chirurgisches Instrument (10) mit einem Instrumentengriff (12), einem mit dem Instrumentengriff (12) verbundenen Instrumentenschaft (14), der eine Instrumentenachse definiert, einer mit dem Instrumentenschaft (14) verbundenen abgewinkelten Instrumentenspitze (16) mit einem Arbeitspunkt (18), der nicht auf der Instrumentenachse angeordnet ist, sowie einem an dem Instrumentengriff (12) oder dem Instrumentenschaft (14) angeordneten ersten Lokalisator (24), wobei der erste Lokalisator (24) ausgebildet ist, sechs Freiheitsgrade zu erfassen und als Lagesensor ausgebildet ist und zwei Spulen aufweist und der Instrumentenschaft (14) zwischen dem ersten Lokalisator (24) und dem Arbeitspunkt (18) während eines Einsatzes des chirurgischen Instruments (10) auslenkbar ist und ein zweiter Lokalisator (20) mit Abstand zum ersten Lokalisator (24) und gegenüber diesem näher an dem Arbeitspunkt (18) und in einem zur Instrumentenspitze benachbarten Bereich des Instrumentenschaftes (14) angeordnet ist,
**wobei** der zweite Lokalisator (20) ausgebildet ist, nur fünf Freiheitsgrade zu erfassen und als Lagesensor ausgebildet ist und eine Spule aufweist, wobei der zweite Lokalisator (20) kleinere Abmessungen als der erste Lokalisator (24) aufweist.

2. Chirurgisches Instrument nach Anspruch 1,
**dadurch gekennzeichnet, dass** Instrumentengriff (12) und Instrumentenschaft (14) lösbar aneinander anordenbar sind.

3. Chirurgisches Instrument nach einem Ansprüche 1 oder 2,
**dadurch gekennzeichnet, dass** der zweite Lokalisator (20) einen Durchmesser von weniger als 0,5 mm, bevorzugt von weniger als 0,4 mm aufweist.

4. Chirurgisches Instrument nach einem der vorangehenden Ansprüche,
**dadurch gekennzeichnet, dass** der erste Lokalisator (24) mindestens einen Reflektor für Licht- und/oder Schallwellen aufweist.

5. Chirurgisches Instrument nach einem der vorangehenden Ansprüche,
**dadurch gekennzeichnet, dass** der Instrumentenschaft (14) eine größere Biegsamkeit als der Instrumentengriff (12) aufweist.

6. Chirurgisches Instrument nach einem der vorangehenden Ansprüche,
**dadurch gekennzeichnet, dass** die Instrumentenspitze (16) zwischen dem zweiten Lokalisator (20) und dem Arbeitspunkt (18) eine geringere Biegsamkeit als der übrige Teil der Instrumentenspitze (16) aufweist.

7. Chirurgisches Instrument nach einem der vorangehenden Ansprüche,
**dadurch gekennzeichnet, dass** das chirurgische Instrument (10) eine Speichereinheit (22) zum Speichern von Relativpositionsdaten des zweiten Lokalisators (20) relativ zum ersten Lokalisator (24) und/oder zum Speichern von Relativpositionsdaten des Arbeitspunkts (18) relativ zum zweiten Lokalisator (24) und/oder zum Speichern von Relativpositionsdaten des Arbeitspunkts (18) relativ zum ersten Lokalisator (24) aufweist.

8. Chirurgisches Instrument nach einem der vorangehenden Ansprüche,
**dadurch gekennzeichnet, dass** der zweite Lokalisator (20) innerhalb einer Außenkontur des chirurgischen Instruments (10) angeordnet ist.

9. Chirurgisches Instrument nach einem der vorangehenden Ansprüche,
**dadurch gekennzeichnet, dass** der zweite Lokalisator (20) weniger als 2 cm vom Arbeitspunkt (18) entfernt in dem chirurgischen Instrument (10) angeordnet ist.

10. Chirurgisches Instrument nach einem der vorangehenden Ansprüche,
**dadurch gekennzeichnet, dass** der erste Lokalisator (24) und der zweite Lokalisator (20) derart an dem chirurgischen Instrument (10) angeordnet sind, dass ein Lageerfassungssystem die Art des verwendeten chirurgischen Instruments (10) aufgrund dem Lageerfassungssystem bereitgestellter Kennwerte über chirurgische Instrumente (10) und die jeweilige Anordnung des ersten Lokalisators (24) und des zweiten Lokalisators (20) an dem chirurgischen Instrument (10) erkennt.

11. Lageerfassungssystem zur Ermittlung der Lage von chirurgischen Instrumenten (10), mit einer Auswerteeinheit (28) zum Ermitteln der Lage von in dem Lageerfassungssystem angeordneten Lokalisatoren (20, 24, 44) und einer Anzeigeeinheit (30) zur lagegetreuen Darstellung des chirurgischen Instruments (10), **gekennzeichnet durch** ein chirurgisches Instrument (10) nach einem der Ansprüche 1 bis 10.

## Claims

1. Surgical instrument (10) comprising an instrument handle (12), an instrument shaft (14) connected to the instrument handle (12), the instrument shaft defining an instrument axis, an angled instrument tip (16) that is connected to the instrument shaft (14) and has an operating point (18) that is not located on the instrument axis, as well as a first locator (24) arranged on the instrument handle (12) or the instrument shaft (14), wherein the first locator (24) is designed to detect six degrees of freedom and configured as a position sensor, and has two coils,
and the instrument shaft (14) is deflectable between the first locator (24) and the operating point (18) when the surgical instrument (10) is in use, and a second locator (20) is arranged at a distance from the first locator (24) and, in relation to the latter, closer to the operating point (18) and in an area of the instrument shaft (14) that is adjacent to the instrument tip,
wherein the second locator (20) is designed to detect only five degrees of freedom and configured as a position sensor, and has one coil, with the dimensions of the second locator (20) being smaller than those of the first locator (24).

2. Surgical instrument according to claim 1,
**characterized in that** the instrument handle (12) and instrument shaft (14) can be arranged next to each other in a detachable manner.

3. Surgical instrument according to claim 1 or 2,
**characterized in that** the second locator (20) has a diameter of less than 0.5 mm, preferably less than 0.4 mm.

4. Surgical instrument according to one of the above claims,
**characterized in that** the first locator (24) has at least one reflector for light waves and/or sound waves.

5. Surgical instrument according to one of the above claims,
**characterized in that** the instrument shaft (14) has a greater flexibility than the instrument handle (12).

6. Surgical instrument according to one of the above claims,
**characterized in that** the instrument tip (16) has a lower flexibility between the second locator (20) and the operating point (18) than the remaining part of the instrument tip (16).

7. Surgical instrument according to one of the above claims,
**characterized in that** the surgical instrument (10) features a memory unit (22) for storing relative position data of the second locator (20) relative to the first locator (24) and/or for storing relative position data of the operating point (18) relative to the second locator (24) and/or for storing relative position data of the operating point (18) relative to the first locator (24).

8. Surgical instrument according to one of the above claims,
**characterized in that** the second locator (20) is arranged within an outer contour of the surgical instrument (10).

9. Surgical instrument according to one of the above claims,
**characterized in that** the second locator (20) is arranged less than 2 cm from the operating point (18) within the surgical instrument (10).

10. Surgical instrument according to one of the above claims,
**characterized in that** the first locator (24) and the second locator (20) are arranged on the surgical instrument (10) in such a way that a position detection system identifies the type of surgical instrument (10) used based on the characteristic values regarding surgical instruments (10) provided to the position detection system and the respective arrangement of the first locator (24) and the second locator (20) on the surgical instrument (10).

11. Position detection system for determining the position of surgical instruments (10), comprising an evaluation unit (28) for determining the position of locators (20, 24, 44) arranged in the position detection system and a display unit (30) for displaying the surgical instrument (10) in its precise position, **characterized by** a surgical instrument (10) according to one of claims 1 through 10.

## Revendications

1. Instrument (10) chirurgical comprenant un manche (12) d'instrument, un fût (14) d'instrument, qui est relié au manche (12) d'instrument et qui définit un axe de l'instrument, une pointe (16) d'instrument coudée reliée au fût (14) de l'instrument et ayant un point (18) de travail, qui n'est pas sur l'axe de l'instrument, ainsi qu'un premier localisateur (24) disposé sur le manche (12) de l'instrument ou sur le fût (14) de l'instrument, dans lequel le premier localisateur (24) est constitué pour détecter six degrés de liberté et est constitué en capteur de position et a deux bobines et le fût (14) de l'instrument peut, pendant une utilisation de l'instrument (10) chirurgical, être dévié entre le premier localisateur (24) et le point (18) de travail et un deuxième localisateur (20) est disposé à distance du premier localisateur (24) et, par rapport à celui-ci, en étant plus près du point (18) de travail et dans une partie du fût (14) de l'instrument voisine de la pointe de l'instrument, dans lequel le deuxième localisateur (20) est constitué pour ne détecter que cinq degrés de liberté et est constitué en capteur de position et a une bobine, le deuxième localisateur (20) ayant des dimensions plus petites que le premier localisateur (24).

2. Instrument chirurgical suivant la revendication 1, **caractérisé en ce que** le manche (12) de l'instrument et le fût (14) de l'instrument peuvent être disposés l'un contre l'autre de manière à pouvoir être détachés.

3. Instrument chirurgical suivant l'une des revendications 1 ou 2,
**caractérisé en ce que** le deuxième localisateur (20) a un diamètre de moins de 0,5 mm, de préférence de moins de 0,4 mm.

4. Instrument chirurgical suivant l'une des revendications précédentes,
**caractérisé en ce que** le premier localisateur (24) a au moins un réflecteur d'ondes lumineuses et/ou sonores.

5. Instrument chirurgical suivant l'une des revendications précédentes,
**caractérisé en ce que** le fût(14) de l'instrument a une souplesse plus grande que le manche (12) de l'instrument.

6. Instrument chirurgical suivant l'une des revendications précédentes,
**caractérisé en ce que** la pointe (16) a, entre le deuxième localisateur (20) et le point (18) de travail, une souplesse plus petite que la partie restante de la pointe (16) de l'instrument.

7. Instrument chirurgical suivant l'une des revendications précédentes,
**caractérisé en ce que** l'instrument (10) chirurgical a une unité (22) de mise en mémoire, pour mettre en mémoire des données de position relatives du deuxième localisateur (20) par rapport au premier localisateur (24) et/ou pour mettre en mémoire des données de position relatives du point (18) de travail par rapport au deuxième localisateur (24) et/ou pour mettre en mémoire des données de position relatives du point (18) de travail par rapport au premier localisateur (24).

8. Instrument chirurgical suivant l'une des revendications précédentes,
**caractérisé en ce que** le deuxième localisateur (20) est disposé à l'intérieur d'un contour extérieur de l'instrument (10) chirurgical.

9. Instrument chirurgical suivant l'une des revendications précédentes,
**caractérisé en ce que** le deuxième localisateur (20) est disposé dans l'instrument (10) chirurgical en étant éloigné de moins de 2 cm du point (18) de travail.

10. Instrument chirurgical suivant l'une des revendications précédentes,
**caractérisé en ce que** le premier localisateur (24) et le deuxième localisateur (20) sont disposés sur l'instrument (10) chirurgical, de manière à ce qu'un système de relevé de position détecte le type de l'instrument (10) chirurgical utilisé sur la base de valeurs caractéristiques, mises à disposition du système de relevé de position, des instruments (10) chirurgicaux et de l'agencement respectif du premier localisateur (24) et du deuxième localisateur (20) sur l'instrument (10) chirurgical.

11. Système de relevé de position pour déterminer la position d'instruments (10) chirurgicaux, comprenant une unité (28) d'exploitation pour déterminer la position de localisateurs (20, 24, 44) disposés dans le système de relevé de position et une unité (30) d'affichage pour la représentation fidèle en position de l'instrument (10) chirurgical,
**caractérisé par** un instrument (10) chirurgical suivant l'une des revendications 1 à 10.
